(19) **Europäisches Patentamt**
**European Patent Office**
**Office européen des brevets**

(11) **EP 4 643 893 A1**

(12) # EUROPEAN PATENT APPLICATION

(43) Date of publication:
**05.11.2025 Bulletin 2025/45**

(21) Application number: **24173587.7**

(22) Date of filing: **30.04.2024**

(51) International Patent Classification (IPC):
***A61L 27/36*** *(2006.01)*

(52) Cooperative Patent Classification (CPC):
**A61L 27/3608; A61L 27/3612; A61L 27/3687;
A61L 27/3691;** A61L 2430/02; A61L 2430/06;
A61L 2430/38

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Industrie Biomediche Insubri S.A.**
**6805 Mezzovico-Vira (CH)**

(72) Inventors:
• **PERALE, Giuseppe**
  **6925 Gentilino in Collina d'Oro (CH)**

• **PERTICI, Gianni**
  **6948 PORZA (CH)**
• **ORLANDO, Luca**
  **20149 Milano (IT)**
• **VARESTI, Arnaldo Emilio**
  **22070 GRANDATE (CO) (IT)**
• **OVREBO, Oystein**
  **1176 Oslo (NO)**

(74) Representative: **Gregorj S.r.l.**
**Via L. Muratori, 13/b**
**20135 Milan (IT)**

(54) **A COLD-CLEANING PROCESS FOR DERIVING A DECELLULARIZED GRAFT TISSUE TO BE USED AS A MEDICAL DEVICE, OR AS A RAW MATERIAL**

(57) The present invention deals with a cold-cleaning process for deriving a decellularized bone tissue sample or decellularized osteochondral tissue sample to be used as a medical device, or as a raw material.

**Description**

**Technical field of the invention**

The present invention relates to a cold-cleaning process for deriving a decellularized bone tissue sample or decellularized osteochondral tissue to the field of dental and orthopaedic tissue healing and improvement.

Said process comprises cleaning steps, each step combining mechanical and chemical cleaning treatm ents, carried out at standard pressure and a low temperature, of a biological material sample, selected from the group comprising bone tissue sample/s or osteochondral tissue sample/s, of animal origin, such as bovine, equine, and porcine, or from humans both living and cadavers.

The cold-cleaning process according to the present invention has been optimized to remove bone marrow, any potential prions, kill bacteria and inactivate virus, and effective removal of lipids, pyrogens, micro-organism and/or pathogens, while maintaining a polycrystalline mineral structure and limiting damage to the collagen phase to produce a decellularized bone tissue sample or decellularized osteochondral tissue sample so obtainable, preferably obtained. The decellularized bone tissue sample or decellularized osteochondral tissue sample so obtainable, preferably obtained according to the cold-clean process object of the present invention can be used as a medical device, or used as a raw material in further processing.

**Background**

Bone xenografts are animal derived material used for regenerating of tissues that are formed by ossification or calcification, such as bone and teeth. Simultaneously, an osteochondral graft combines the osseous phase of the a long bone with the cartilage at the end in an integrated, phased structure. A cleaning process is a process where the tissue from an animal is refined, typically by removal of components such as bone marrow and cells, to obtain a material suitable for implantation in the human body where it then stimulates bone regeneration and/or replace damaged articular cartilage. The need for bone regeneration is diverse, including dental, orthopaedic and spinal application. A commonality however is that bone grafts quickly need to integrate with the native bone, not provoke any chronic inflammatory response or transfer of pathogenesis, and it need to immediately provide adequate mechanical support. In the case of dental application, e.g. sinus lift, grafts are typically designed such that a dental implant can be inserted 3 months after the grafting procedure. For orthopaedic application the graft must, in combination with any support plates or screws, provide enough mechanical support to prevent movement that can inhibit fracture healing, e.g. for tibial plateau fractures [1]. Thus, for these two applications mechanical integrity is vital. For spinal fusion, a titanium or PEEK cage is typically used to obtain adequate spacing, reducing the requirements for mechanical strength. In all cases the biological performance is crucial as quicker bone growth will increase patient satisfaction and can reduce healthcare costs. An ideal bone graft should exhibit osteoconductive properties, have a high porosity, and be able to take loading [2]. However, as pointed out by Schickert and colleagues [3], the biological and mechanical requirements are often contradictory for loading-application, thus a trade-off between these properties are required during the manufacturing process.

For animal derived tissues the European Medical Device Regulations (MDR - 2017/745) and regulation 2012/722 on use of medical devices of animal origin requires the tissue to be sourced in a manner that minimize risk of pathogens transmission by implementing validated methods for the inactivation or elimination of these pathogens during the manufacturing [4, 5]. In terms of bovine sourced grafts the main concern is *bovine spongiform encephalopathy* (BSE), which the European Pharmacopoeia suggests can be inactivated or eliminated using sodium hydroxide, sodium hypochlorite, or temperatures above 133 °C [6]. Many producers choose to sinter their grafts at temperatures typically ranging between 650-1200 °C, which does indeed remove any pathogens, but it also makes a xenograft anorganic by removing the collagen and changes the mineral structure to a high degree of crystallinity. This is the case with Bio-Oss®, the market leading bone graft for dental applications in Europe. Native bone consists of hydroxyapatite and collagen, where the mineral crystals are nucleated and grows out of the collagen yielding a composite material that is hierarchical organized down to a nanoscale [7]. This provides the combined high mechanical stiffness and high fracture toughness, where the collagen fibres is essential for the latter property [8]. The fracture toughness prevents catastrophic failure of the graft when exposed to loading, that a bone graft will typically experience after implantation. Although many methods high temperature sintering methods [9], there are also methods using biologically viable temperatures, maintaining the collagen and thereby the fracture toughness. Considering these low temperature methods for preparation of allografts (human derived) and xenografts, most cleaning procedures starts with treatments to remove the bone marrow components from the structure. Over the last decade a series of articles have started their cleaning with 1 % Triton X-100 (Polyethylene glycol tert-octylphenyl ether), a nonionic surfactant [10-15]. However, Triton X-100 has been put on the SVHC (Substances of Very High Concern) list according to the REACH (Registration, Evaluation, Authorisation and Restriction of Chemicals) regulation by the European Chemical Agency due to its endocrine disrupting properties and is therefore not suitable for bone cleaning. The use of 3% hydrogen peroxide and 70% ethanol for further removal of fat has also been reported [11].

Other agents commonly used includes sodium hydroxide for viral and prion inactivation, and oxidating agents such as hydrogen peroxide for elimination of cells and debris, but both are recognized to have determinantal effect on the mineral and collagen structure of bone [16]. Therefore, the concentration and processing time should be kept to a minimum.

Tutoplast xenograft by Tutogen Gmbh utilizes a series of steps including $H_2O_2$, NaOH, and acetone [16]. The process has been described in detail by Christoph Schoepf [17]; 1) removal of lipids with an ultrasonic acid bath, which is also claimed to reduce any prion load by two-log and inactivating viruses. 2) rinse in baths of hyperosmotic salt water, which erupts the cell membrane, exposing any intracellular viruses, and wash out cell debris, including for bacteria. 3) hydrogen peroxide is then used to eliminate soluble proteins, viruses, and bacterial spores through oxidation. 4) A final acetone bath is used to reassure removal of prions and virus inactivation. Thereafter the graft is dehydrated using vacuum extraction before it is sterilized using low dose gamma irradiation (17.8 kGy to 20.1 kGy). Typically, for dental application, allografts are consumed in the American market and bovine xenografts in the European market

[2], and it can be speculated that Tutogen Gmbh use their method to produce market specific products. Bansal and colleagues [18] were able to show that xenograft blocks made with the Tutoplast method were able to support fracture union in tibia plateau fracture while maintaining mechanical integrity, demonstrating the importance of maintaining the collagen phase. Indeed, in an elder patient group (average age of 74, n=19) they observed an average union time of 20 weeks and an average collapse of 4 mm.

Accordingly, there was a long felt need to develop an adequate cleaning process in order to achieve a proper graft device of human or animal origin having suited histological properties (to remove bone marrow, any potential prions, kill bacteria and inactivate virus, and effective removal of lipids, pyrogens, micro-organism and/or pathogens) while maintaining a polycrystalline mineral structure and limiting damage to the collagen phase.

### Summary of the invention

Surprisingly and unexpectedly, the Applicant has developed an optimized cleaning process to remove bone marrow, any potential prions, kill bacteria and inactivate virus, while maintaining a polycrystalline mineral structure and limiting damage to the collagen phase.

It is an object of the present invention a cleaning process to produce a decellularized bone tissue sample or decellularized osteochondral tissue sample, as well as a decellularized bone tissue sample or decellularized osteochondral tissue sample so obtained and said decellularized bone tissue sample or decellularized osteochondral tissue sample subsequently washed, dried and sterilized for use as a medical device.

Accordingly, an object of the present invention is:

A cleaning process to produce a decellularized bone tissue sample or decellularized osteochondral tissue sample, comprising cleaning steps, each step combining mechanical and chemical cleaning treatments, carried out at standard pressure and a low temperature, of a biological material sample, said process comprising of:

a) Providing a biological material sample selected from the group comprising bone tissue sample/s or osteochondral tissue sample/s, as a shaped block/s, or chip/s, respectively;

b) Treating said biological material sample/s at a series of cleaning steps at standard pressure and low temperature, preferably no more than 37 °C, each step combining mechanical and chemical cleaning treatments, as follows:

i. Soaking said biological material sample/s into distilled water as a chemical cleaning treatment in combination with centrifugation and sonication of said biological material sample as a mechanical cleaning treatments;

ii. Soaking biological material sample/s treated/obtained according to step bi) into Slightly alkaline solution (pH=8.0-10.0) as a chemical cleaning treatment in combination with centrifugation and sonication of said biological material sample/s as a mechanical cleaning treatments;

iii. Soaking biological material sample/s treated/obtained according to step bii) into organic solvent as a chemical cleaning treatment in combination with centrifugation and sonication of said biological material sample/s as a mechanical cleaning treatments;

iv. Soaking biological material sample/s treated/obtained according to step biii) into Alkaline solution (pH=10.0-14.0) as a chemical cleaning treatment in combination with centrifugation and sonication of said biological material sample/s as a mechanical cleaning treatments;

v. Reacting biological material sample/s treated/obtained according to step biv) with Oxidating agent as a chemical cleaning treatment in combination with centrifugation and sonication of said biological material sample as a mechanical cleaning treatments.

Advantageously, and further preferred, according to any of the embodiments of the cleaning process according to the present invention, the low temperature is from 20°C to 45°C, preferably from 23 °C to 42 °C, more preferably no more than 37°C.

Advantageously, and further preferred, according to any of the embodiments of the cleaning process according to the present invention, the organic solvent is selected from the group comprising: Polar aprotic solvent, such as DCM, Acetone, Acetonitrile, ethyl acetate, pyridine, tetrahydrofuran); halogen-alkane, such as Dichloroethane, Trichloroethane, Chloroform, Carbon tetrachloride, Carbon disulfide ; alcohol, such as Ethanol, Propanol, Methanol, Butanol; ether, such as Diethyl ether; alkane, such as Pentane, Hexane.

Advantageously, and further preferred, according to any of the embodiments of the cleaning process according to the present invention, the Oxidating agent is selected from the group comprising: $H_2O_2$, Peracetic acid, $Na_2O_2$ $MgO_2$, $CaO_2$, $K_2O_2$ $Na_2CO_3$;

Advantageously, and further preferred, according to any of the embodiments of the cleaning process according to the present invention, the centrifugation is for at least 1 minute and less than 15 minutes, at a relative centrifugal force of at least 3000xg and less than 9000xg (xg: x times gravity);

Advantageously, and further preferred, according to any of the embodiments of the cleaning process according to the present invention, the mechanical cleaning treatment, as a solid-liquid extraction treatment is selected from centrifugation extraction, sonication, water jet, hydraulic pressure, super critical fluid extraction;

Advantageously, and further preferred, according to any of the embodiments of the cleaning process according to the present invention, the bone tissue sample/s or decellularized osteochondral tissue sample/s is/are of animal origin, such as bovine, equine, and porcine, or from humans both living and cadavers;

As further objects of the present invention there are:

- a decellularized bone tissue sample obtainable, preferably obtained, by the process according to any one of the above mentioned embodiments thereof;
- a decellularized osteochondral tissue sample obtainable, preferably obtained, by the process according to any one of the embodiments 1 to 7;
- a decellularized bone tissue sample obtainable, preferably obtained, by the process according to any one of the embodiments thereof, and subsequently washed, dried and sterilized for use as a medical device;
- a decellularized osteochondral tissue sample obtainable, preferably obtained, by the process according to any one of the embodiments thereof, and subsequently washed, dried and sterilized for use as a medical device;
- a decellularized bone tissue sample obtainable, preferably obtained, by the process according to any one of the embodiments thereof, and subsequently washed, dried and sterilized for use as a base matrix of a bone implant matrix;
- a decellularized bone tissue sample obtainable, preferably obtained, by the process according to any one of the embodiments thereof, and subsequently washed, dried and sterilized for use as a xenograft/allograft material for filling of bone voids and to induce bone regeneration in dental and orthopaedic bone defects, wherein the dental bone defects relates to the jaw and other osseotissues in the oral orifice and wherein the orthopaedic bone defects relates to all bone tissue defects including the skull and flat bone, long bone, short bone, and vertebrae/vertebras;
- a decellularized osteochondral tissue sample obtainable, preferably obtained, by the process according to any one of the embodiments thereof, and subsequently washed, dried and sterilized for use as a xenograft/allograft material to replace damaged cartilage in joints including the knees, hip, elbow, shoulders, wrists, fingers, toes, and facet joints, as well as replacing cartilage at the end of vertebrae/vertebras, with the osseous phase of the osteochondral graft having the ability to integrate with the surrounding native tissue;
- a decellularized bone tissue sample or decellularized osteochondral tissue sample obtainable, preferably obtained, by the process according to any one of the embodiments thereof, and subsequently washed, dried and sterilized for use as a xenograft/allograft material to induce ectopic bone formation, preferably in a spinal fusion procedure and/or ankle fusion procedures.

The cold-cleaning process according to the present invention has been optimized to remove bone marrow, any potential prions, kill bacteria and inactivate virus, and effective removal of lipids, pyrogens, micro-organism and/or pathogens, while maintaining a polycrystalline mineral structure and limiting damage to the collagen phase to produce a decellularized bone tissue sample or decellularized osteochondral tissue sample so obtainable, preferably obtained. The decellularized bone tissue sample or decellularized osteochondral tissue sample so obtainable, preferably obtained according to the cold-clean process object of the present invention can be used as a medical device Among the Objects of the present invention is a cold-cleaning process for deriving a bone xenograft, preferably bovine bone xenograft for bone regeneration.

Said cold-cleaning process comprises cleaning steps, each step combining mechanical and chemical cleaning treatments, carried out at standard pressure and a low temperature, preferably not higher than 37°C, wherein the bone marrow of bone, preferably bovine bone, is successfully removed, leaving a clean bone xenograft without residual blood, lipids, nor cell debris.

The derived bone xenograft obtainable, preferably obtained, by the process according to the present invention after sterilisation, preferably with β-ray sterilisation, can be used as an implantable material for bone regeneration, or the derived bone xenograft obtainable, preferably obtained by the process according to the present invention can be further modified, preferably after its sterilisation, preferably through coating with a polymeric material, with or without proline-rich peptides, to form a xenohybrid material, or the derivation of an osteochondral implant, preferably obtained by the process according to the present invention, that can replace damaged articulate cartilage while integrating in the surrounding bone of the implant..

The material characterization of the cleaned bone xenograft, preferably the cleaned bovine bone xenograft, obtained according to the cold-cleaning process of the present invention, confirms that the xenograft has suitable morphological properties with a high porosity (>70%) while maintaining adequate mechanical properties.

The evidence shows that the cold-cleaning process according to the present invention provides a xenograft which maintains a native-like bone structure with limited alteration to the calcium phosphate-collagen structure and a low crystallinity, being to be clinically favourable compared to inorganic xenograft that exhibits a high degree of crystallinity (sintered xenograft) The evidence further shows that the cold-cleaning process according to the present invention removes the bone marrow and decellularize the structure, effectively removing lipids, viruses, bacteria, pyrogens, prions, micro-organisms, and/or pathogens. The evidence also shows that the cold-cleaning process according to the present invention provides a graft structure that is not cytotoxic.

## List of figures

Particular embodiments of the invention are described in detail herein below, as a way of example and not limited to, with reference to the attached figures, wherein:

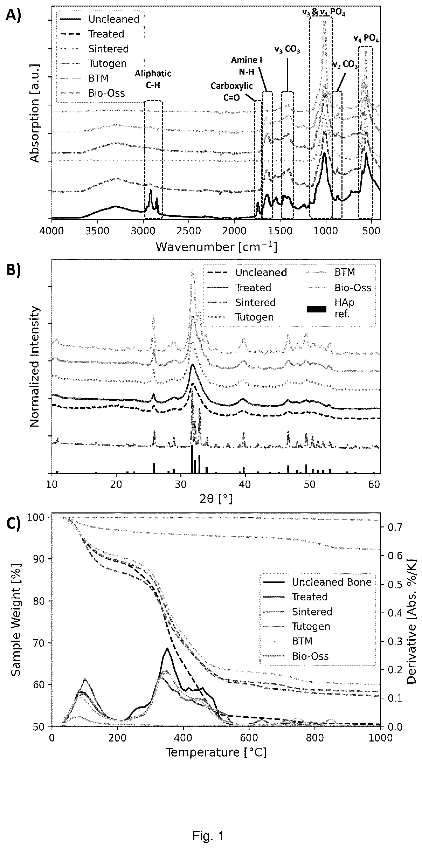

- Figure 1: A) ATR-FTIR plot of the treated (bone xenograft, or decellularized bone tissue, derived from the cold-cleaning process according to the present invention) and sintered (xenograft graft sintered at 1100 °C) grafts compared to untreated bone and benchmarked against three commercial bone grafts prepared using allogenic bone with a low-temperature process (BTM®), xenograft bone with a low-temperature process (Tutogen), and xenograft bone with sintering (Bio-Oss®). B) XRD patterns for the samples, with reference hexagonal hydroxyapatite as reference material. The Sintered graft and Bio-Oss® displayed at peak at 37.7 degrees, which is believed to be calcium oxide from calcification of calcium carbonate. C) TGA plot for the samples, dotted lines represent the wt.% and the full line is the derivative with respect to temperature;

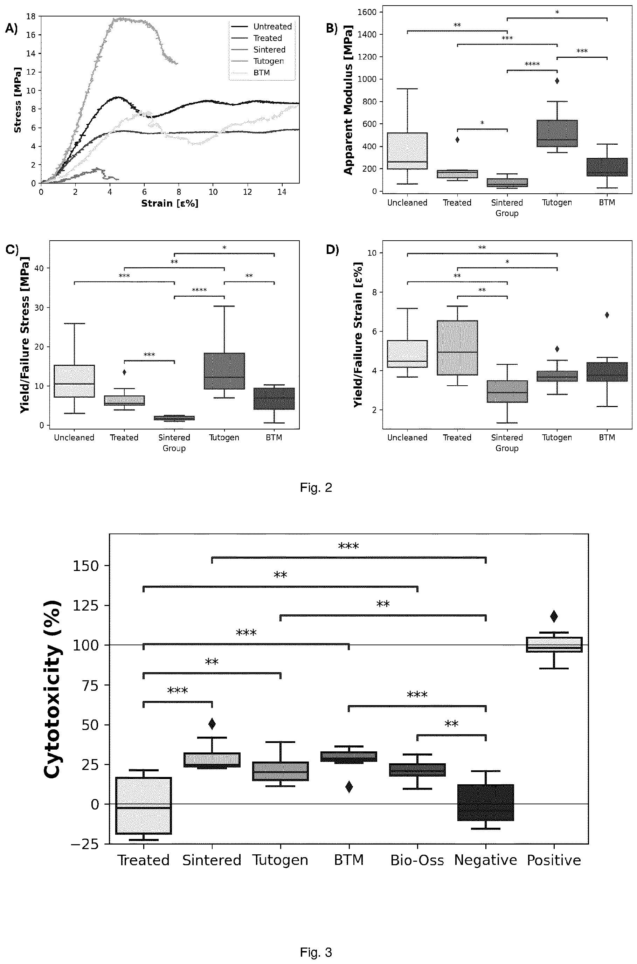

- Figure 2: Results from uniaxial compressive testing. A) Representative stress-strain plot. B). Apparent modulus, C) Yield stress (failure Strain for Sintered), D) Yield Strain (failure strain for Sintered) n = [Untreated: 8; Treated: 8; Sintered: 8; Tutogen; 16; BTM: 7], *p<0.05, **p<0.01, ***p<0.001, ****p<0.0001.
- Figure 3: Cytotoxic effect of the graft exudates on MC3T3-E1 cell line conducted with a LDH array after 48 hours incubation. The ISO standard 10993-5 recommends a cytotoxicity below 30% for the graft to be biocompatible. Surprisingly, all grafts with exception of the Treated had a cytotoxicity approaching 30%, while the Treated graft displayed a negative cytotoxicity;

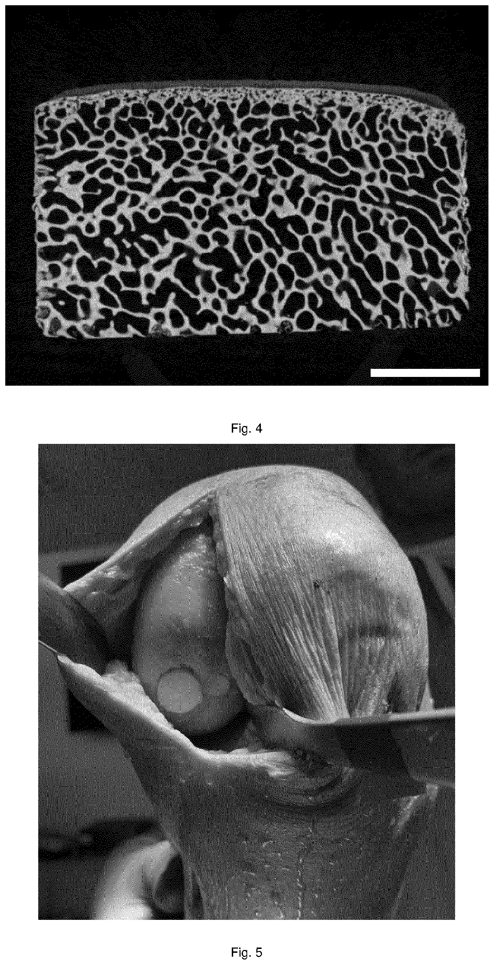

- Figure 4: Micro computed tomography reconstruction of the decellularised osteochondral sample obtained by the cold-cleaning process according to the present invention. The cross-section illustrates that native-like osteochondral properties are maintained, with the articular cartilage intact on the top where it is still attached to the cortical bone tidemark. When going further down into the structure the bone changes from compact cortical to cancellous bone, with increasing porosity and pore size further into the structure. The cartilage had been stained with 0.3% Phosphotungstic acid in PBS for 24 hours prior to the imaging. Scalebar: 5 mm;
- Figure 5: Two decellularised osteochondral samples of varying diameter obtained by the cold-cleaning process implanted in a human knee joint to replace the native articular cartilage;

## Definitions

In the present context:

- the xenograft term refers to a tissue material derived from a mammal different than human used for implantation to obtain a medical effect;
- the bone xenograft as described above refers to tissue material intended to treat bone defects in humans and animals, typically bone tissue of animal origin;
- the Autograft term refers to tissue material transplanted with a human;
- the Allograft term refers to tissue material transplanted between humans;

- the Treated/treated graft term refers to decellularized xenograft obtained by the cold-cleaning process according to the present invention;
- the Sintered/sintered graft term refers to decellularized xenograft obtained by the cold-cleaning process according to the present invention that has further undergone sintering;
- the term Untreated refers to a biological material sample before phase b) of the cold-cleaning process according to the present invention;
- the term Tutogen refers to commercial control as a xenograft prepared using a low-temperature chemical cleaning process known as the 'Tutoplast method';
- the term BTM® refers to commercial control as an allograft prepared using a low-temperature chemical cleaning process, not according to the present invention;
- the term Bio-Oss® refers to commercial control as a sintered xenograft.

The term Bone regeneration - as used herein refers to the replacement of compromised bone and/or filling of bone void volume with native bone. This can be done naturally by the body itself, or, as typically needed for larger defects, through the implantation of a temporary support structure, a graft, that facilitates the regeneration of the missing bone, meaning that the graft gets absorbed and replaced by native bone.

The term of a decellularized osteochondral tissue sample of human or animal origin, preferably bovine origin, as used herein refers to a decellularized osteocondral graft that can be used as an implant in human and animal joints to replace damaged and/or degraded articular cartilage, with the subchondral part being anchored in and integrated with the surrounding native bone.

The decellularized osteochondral graft according to the present invention has a layer of non-mineralised collagen. The decellularized osteochondral graft according to the present invention comprises a combination of two distinct region: the osteo-region which is a bone region, coated by a chondral region which is the layer of cartilage that is attached to the bone, i.e. a distinct layer of 1-2 mm thickness.

The term "biocompatible" as used herein refers to causing no clinically relevant tissue irritation, injury, toxic reaction, or immunological reaction to living tissue.

The term "cell" is used herein to refer to the structural and functional unit of living organisms and is the smallest unit of an organism classified as living.

The term "prion" is used herein to refer to an infectious protein particle that lacks genetic material and can cause abnormal protein folding.

The term "virus" is used herein to refer to a microscopic infectious agent that is not classified as a living organism in the traditional sense.

Viruses consist of genetic material, such as DNA or RNA, enclosed in a protein coat. They lack the cellular structure and metabolic processes typically associated with living organisms and are incapable of independent replication or metabolism.

The term "bone marrow" is used herein to refer to the spongy and fatty tissue residing within the cavities of bones, notably in the long bones, such as the femur, tibia, and pelvis, as well as in flat bones like the sternum and the skull. This specialized tissue functions as the primary site for hematopoiesis, where blood cells, including red blood cells (erythrocytes), white blood cells (leukocytes), and platelets (thrombocytes), are produced and regulated.

The term "compatible" as used herein means that components of a composition are capable of being combined with each other in a manner such that there is no interaction that would substantially reduce the efficacy of the composition under ordinary use conditions.

The term "component" as used herein refers to a constituent part, element, or ingredient.

In the current context, 'M' refers to the molar concentration of a specific component, 'xg' refers to a number accelerated by the gravitational constant ($9.81\ ms^{-2}$), while 'gr' after a number refers to grams

In the present context, the term shaped block refers to any three-dimensional portion, whether of a regular shape, such as those of a geometric type (cubes, parallelepipeds, cylinders, pyramids, etc.) or of an irregular shape.

In the present context, with the term treated/cleaned/derived graft/xenograft according to the present invention is meant "a decellularized bone tissue sample or decellularized osteochondral tissue sample obtainable, preferably obtained, by the process according to any one of the embodiments of the present invention".

The term "sonication" as used herein refers to a sonication treatment within the standard meaning of sonication, i.e. is the act of applying sound energy to agitate particles in a sample, in particular the sound waves migrate through a medium, inducing pressure variations and cavitations that grow and collapse, transforming the sound waves into mechanical energy, in particular to speed dissolution, wherein the power of frequencies to be applied for the sonication treatment according to the present invention is due to the nature of the biological material sample to be traded, i.e. a bone tissue or osteochondral tissue.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges which may independently be included in the smaller ranges is also encompassed within the invention, subject to any specifically excluded limit in the stated range. Where the stated range includes one or both of the limits, ranges excluding both of those included limits are also included in the invention.

It is to be understood that the terminology used herein is for describing particular embodiments only and is not intended to limit the scope of the present invention which will be defined by the appended claims.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, exemplary methods and materials have been described. All publications mentioned herein are incorporated to disclose and describe the methods and/or materials in connection with which the publications are cited.

## Detailed description of the invention

It is an object of the present invention a cleaning process to produce a decellularized bone tissue sample or decellularized osteochondral tissue sample, as well as a decellularized bone tissue sample or decellularized osteochondral tissue sample so obtained and said decellularized bone tissue sample or decellularized osteochondral tissue sample subsequently washed, dried and sterilized for use as a medical device.

Among the preferred embodiments of the cleaning process to produce a decellularized bone tissue sample or decellularized osteochondral tissue sample, according to the present invention, there are:

1) A cleaning process to produce a decellularized bone tissue sample or decellularized osteochondral tissue sample, comprising cleaning steps, each step combining mechanical and chemical cleaning treatments, carried out at standard pressure and a low temperature, of a biological material sample, said process comprising of:

a) Providing a biological material sample selected from the group comprising bone tissue sample/s or osteochondral tissue sample/s, as a shaped block/s, or chip/s, respectively;

b) Treating said biological material sample/s at a series of cleaning steps at standard pressure and low temperature, preferably no more than 37 °C, each step combining mechanical and chemical cleaning treatments, as follows:

i. Soaking said biological material sample/s into distilled water as a chemical cleaning treatment in combination with centrifugation and sonication of said biological material sample as a mechanical cleaning treatments; preferably a mass-to-volume ratio between the biological sample material and water is from 1 gr to 30ml, preferably from 1 gr to 25 ml, more preferably from 1 gr to 20 ml;

ii. Soaking biological material sample/s treated/obtained according to step bi) into Slightly alkaline solution (pH=8.0-10.0) as a chemical cleaning treatment in combination with centrifugation and sonication of said biological material sample/s as a mechanical cleaning treatments;

iii. Soaking biological material sample/s treated/obtained according to step bii) into organic solvent as a chemical cleaning treatment in combination with centrifugation and sonication of said biological material sample/s as a mechanical cleaning treatments; preferably the soaking in the organic solvent, such as polar aprotic solvent, halogen-alkane, Carbon disulfide , alcohol, ether, alkane, is performed twice followed by washing in water, air dried and centrifuged again;

iv. Soaking biological material sample/s treated/obtained according to step biii) into Alkaline solution (pH=10.0-14.0) as a chemical cleaning treatment in combination with centrifugation and sonication of said biological material sample/s as a mechanical cleaning treatments; preferably the alkaline chemical treatment is followed by a neutralization phase by treating the sample in a buffer solution and washed with water always in presence of the mechanical treatments;

v. Reacting biological material sample/s treated/obtained according to step biv) with Oxidating agent as a chemical cleaning treatment in combination with centrifugation and sonication of said biological material sample as a mechanical cleaning treatments;

so obtaining the decellularized bone tissue sample or decellularized osteochondral tissue sample.

2) Process according to embodiment 1, wherein the low temperature is from 20°C to 45°C, preferably from 23 °C to 42 °C., more preferably no more than 37°C;

3) Process according to embodiments 1 to 2, wherein the organic solvent is selected from the group comprising: Polar aprotic solvent, such as DCM, Acetone, Acetonitrile, ethyl acetate, pyridine tetrahydrofuran halogen-alkane, such as Dichloroethane, Trichloroethane, Chloroform, Carbon tetrachloride, Carbon disulfide ; alcohol, such as Ethanol, Propanol, Methanol, Butanol; ether, such as Diethyl ether; alkane, such as Pentane, Hexane;

4) Process according to embodiments 1 to 3, wherein the Oxidating agent is a solution of an oxidating reagent selected from the group comprising: $H_2O_2$, Peracetic acid, $Na_2O_2$, $MgO_2$, $CaO_2$, $K_2O_2$ $Na_2CO_3$;

5) Process according to embodiments 1 to 4 wherein the centrifugation is for at least 1 minute and less than 15 minutes, at a relative centrifugal force of at least 3000xg and less than 9000xg;

6) Process according to embodiments 1 to 4 wherein the mechanical cleaning treatment, as a solid-liquid extraction treatment is selected from centrifugation extraction, sonication, water jet, hydraulic pressure, super critical fluid extraction;

7) Process according to embodiments 1 to 6 wherein the bone tissue sample/s or decellularized osteochondral tissue sample/s is/are of animal origin, such as bovine, equine, and porcine, or from humans both living and cadavers;

8) Process according to embodiments 1 to 7 further comprising washing the biological material sample/s treated/obtained according to step bvi) by water, preferably followed by centrifugation treatment;

9) Process according to embodiments 1 to 8 further comprising drying the biological material sample/s after being washed with water, drying such as vacuum drying;

10) Process according to embodiments 1 to 9 further comprising sterilization of the biological material sample/s after being dried, sterilization such as electron beam sterilization;

11) Process according to embodiments 1 to 7 further comprising a washing treatment of the biological material sample/s treated/obtained according to step bvi) by water, preferably followed by centrifugation treatment; a drying treatment of the biological material sample/s after being washed with water, drying treatment such as vacuum drying; a sterilization treatment of the biological material sample/s after being dried, sterilization treatment such as electron beam sterilization;

12) Process according to any embodiments 1 to 11 wherein the Oxidating agent comprises from 3 to 33% w/w, preferably from 5 to 15 % w/w of the oxidating reagent.

As further objects of the present invention there are:

- a decellularized bone tissue sample obtainable, preferably obtained, by the process according to any one of the embodiments 1 to 7;
- a decellularized osteochondral tissue sample obtainable, preferably obtained, by the process according to any one of the embodiments 1 to 7;
- a decellularized bone tissue sample obtainable, preferably obtained, by the process according to any one of the embodiments 1 to 7 and subsequently washed, dried and sterilized for use as a medical device;
- a decellularized osteochondral tissue sample obtainable, preferably obtained, by the process according to any one of the embodiments 1 to 7 and subsequently washed, dried and sterilized for use as a medical device;
- a decellularized bone tissue sample obtainable, preferably obtained, by the process according to any one of the embodiments 1 to 7 and subsequently washed, dried and sterilized for use as a base matrix of a bone implant matrix;
- a decellularized bone tissue sample obtainable, preferably obtained, by the process according to any one of the claims 1 to7 and subsequently washed, dried and sterilized for use as a xenograft/allograft material for filling of bone voids and to induce bone regeneration in dental and orthopaedic bone defects, wherein the dental bone defects relates to the jaw and other osseotissues in the oral orifice and wherein the orthopaedic bone defects relates to all bone tissue defects including the skull and flat bone, long bone, short bone, and vertebrae/vertebras;
- a decellularized osteochondral tissue sample obtainable, preferably obtained, by the process according to any one of the claims 1 to 7 and subsequently washed, dried and sterilized for use as a xenograft/allograft material to replace damaged cartilage in joints including the knees, hip, elbow, shoulders, wrists, fingers, toes, and facet joints, as well as replacing cartilage at the end of vertebrae/vertebras, with the osseous phase of the osteochondral graft having the ability to integrate with the surrounding native bone tissue;
- a decellularized bone tissue sample or decellularized osteochondral tissue sample obtainable, preferably obtained, by the process according to any one of the claims 1 to 7 and subsequently washed, dried and sterilized for use as a xenograft/allograft material to induce ectopic bone formation, preferably in a spinal fusion procedure and/or ankle fusion procedures.

One of the essential characteristics of the process according to the present invention, according to any of its embodiments, the slightly alkaline solution in step bii) is used to improve the removal of bone marrow from internal portion of the biological material to the structure, before the organic solvent is used in step biii) to dissolve the remaining fat in the samples.

Preferably, the slightly alkaline solution is an aqueous solution with a pH value ranging from 8.0 to 10.0, preferably from 7.5 to 9.0, more preferably obtained dissolving in water buffer chemical compounds such as: sodium bicarbonate, acetic acid - sodium acetate, ammonium hydroxide - ammonium chloride, ammonium acetate, ammonium carbonate, sodium bicarbonate - sodium carbonate.

As a further essential features of the process according to the present invention, according to any of its embodiments, in step biii) the organic solvent, among those Polar aprotic solvent, such as DCM, Acetone, Acetonitrile, ethyl acetate, pyridine tetrahydrofuran; halogen-alkane, such as Dichloroethane, Trichloroethane, Chloroform, Carbon tetrachloride, Carbon disulfide ; alcohol, such as Ethanol, Propanol, Methanol, Butanol; ether, such as Diethyl ether; alkane, such as Pentane, Hexane is efficient as dissolving the fat, and extracts most of the fat.

However it still leaves a small layer of fat coating the sample.

Therefore, it is a further essential feature of the process according to the present invention in step biv) the use of an alkaline solution to break down the last remaining layer of the fat.

Preferably, the alkaline solution is an aqueous solution with a pH value ranging from 10.0 to 14.0, preferably from 11.5 to 14, more preferably obtained dissolving in water alkaline chemical compounds such as: NaOH, KOH, $Ca(OH)_2$, $Mg(OH)_2$, NHs.In addition to removing fat from bone marrow, both the use of organic solvent as chemical cleaning treatment in step biii) and the use of alkaline solution as chemical cleaning treatment in step biv) are both important for removal of potential pathogen agents.

Lastly, the use of an oxidizing agent as a chemical treatment in step bv) is due to its antiseptic properties to reassure removal of pathogens and it bleaches the samples giving a more pleasant appearance of the decellularized bone tissue sample or decellularized osteochondral tissue sample as a graft.

Since the use of an alkali solution and an oxidizing agent are strongly basic and acidic respectively, preferably the decellularized bone tissue sample or decellularized osteochondral tissue sample so obtainable, or obtained, according to the process of the present invention, said decellularized bone tissue sample or decellularized osteochondral tissue sample are neutralized by a buffer solution before to be washed in water, preferably distilled water.

To improve the extraction of any toxic leachable, the samples are left in the water under stirring. Subsequently, they are dried, such as for 24 h in a vacuum oven. Afterwards the dried samples are packed in double pouches and sterilized using electron-beam irradiation, as this is known for inactivating bacterial and viral load through cleavage of their DNA chains.

In particular, when the decellularized bone tissue sample is an intermediate decellularized xeno-bone graft or an intermediate decellularized bovine bone graft so obtained, it is subsequently washed in water, preferably distilled water, preferably for 2 h under stirring, dried, preferably by vacuum drying at 37 °C and 30 mmHg for 24 h and sterilized, preferably sterilized by β-ray sterilization at 25 kGry, providing bone xenograft, more preferably bovine bone xenograft, ready to be used.

When the biological material sample provided in step a) according to the process of the present invention, is a bone tissue selected from a xeno-bone or bovine bone, they underwent to cutting stage providing blocks/pieces of different size. These pieces are loaded with bone marrow, which needs to be removed for efficient decontamination from potential bacterial, viral or fungal pathogens.

If the xeno-bone or bovine bone are frozen, the initial 1h defrosting in distilled water at 37 °C followed by 10 minutes centrifugation at relative centrifugal force not less of 3000xg (gravity) as the step bi) of the process according to the present invention, of the blocks/pieces of step a), is enough to remove 25% of the initial mass.

Accordingly, as a preferred embodiment of the steps a) and bi) of the process of the present invention, when the biological material sample is of xeno-bone or bovine bone origin, the step a) and bi) are as follows: step a) - providing xeno-bone, preferably bovine bone, preferably cutting xeno-bone, preferably bovine bone into pieces/samples/blocks; step bi) - if necessary, defrost the xeno-bone/bovine bone in water at low temperature, preferably not higher than 37°C, preferably for 1 h under stirring; and centrifuged the xeno-bone/bovine bone, preferably at least twice for 5 min. or once for 10 min., at relative centrifugal force not less than 3000xg.

The following, not limitative, examples describe embodiments of the invention.


**EXAMPLES**


**Example 1: Processing of bovine bone**

A long bone of a young bull, less than 30 months of age, was collected at a butcher. The cortical bone was removed using an electronic saw, leaving an irregular block of porous bone filled with bone marrow, commonly referred to as trabecular bone. The trabecular bone was further cut into smaller sections using the saw. The samples were then processed for evaluation using hyper/hypo osmotic soaks, alkaline soaks, dehydration in organic solvents, and soaks in hydrogen peroxide, and oven drying.

Specific processing of these grafts included storage in, multiple hypo / hyperosmotic soaks (utilizing slightly alkaline solutions, such as sodium bicarbonate solution and distilled water, solvent dehydration using polar aprotic solvent such as dichloromethane, lipid degradation with alkaline solution, such as sodium hydroxide solution, treatment with oxidating agent, such as hydrogen peroxide, and oven drying at 37 ° C.

Immunohistochemistry using primary antibody rabbit polyclonal anti-bovine collagen type I suggested that the bovine bone samples were weakly positive to Collagen I, and no osteocytes were observable in the bone lacunae.

The bone structure was maintained, and there were no signs of residual lipids or cellular debris throughout the bone structure.

## Example 2: Processing of human bone

A long bone of a human cadaver was collected from a bone bank. The cortical bone was removed using an electronic saw,, leaving an irregular block of porous bone filled with bone marrow, commonly referred to as trabecular bone. The trabecular bone was further cut into smaller sections using the saw. The samples were then processed for evaluation using hyper/hypo osmotic soaks, alkaline soaks, dehydration in organic solvents, and soaks in hydrogen peroxide, and oven drying - similar to the process described in Example 1.

The processing yielded an intact trabecular bone structure without cellular debris or adipose tissue, similar to the quality of the bone derived in example 1.

## Example 3: Processing of a bovine osteochondral plug for implantation

An osteochondral plug, meaning a cylindrical tissue piece consisting of a cartilage layer, a cortical bone layer, and a trabecular bone layer fully integrated in a transitionary manner, was extracted from the joint of a young bull, less than 30 months of age.

The samples underwent a similar treatment as described in example 1. This yielded a bone structure free of cellular debris and adipose tissue. The cartilage was maintained, providing a low friction surface once hydrated. The sample was then stained by submergence in 0.3% Phosphotungstic acid in PBS for 24 hours prior to imaging with a micro-computed tomography system to obtain a cross sectional view. When looking at the cross section of the graft, it is characterised by an intact cartilage layer on the top of a layer of cortical bone (compact bone), which transitions to cancellous (porous) bone when moving further down as displayed in Figure 4..

**Example 4:** Characterisation of the properties of a derived bone xenograft and comparison to state-of-the-art

The aim of this example is to demonstrate which properties the graft developed as prepared in example 1, herein described as "Treated", has compared to current state of the art. This was done by also sintering the graft ("Sintered"), to demonstrate the importance of the low-temperature process. Further, it is compared to a commercial bovine xenograft prepared from a cold-temperature cleaning process. "Tutogen", an allograft prepared from a cold-temperature process, "BTM®", and a sintered bovine xenograft, "Bio-Oss®". The physio-chemical properties of these grafts were described in addition to the in vitro cytotoxicity.

## Materials:

All chemicals were ordered in pharmaceutical grade versions from VWR Switzerland. Positive control groups were purchased directly from the supplier, used as supplied and were as follows: BTM® granules of 0.25-1 mm (allograft - IOR Bologna, Italy), Tutogen blocks of 10x10x10 mm$^3$ and granules of 0.25-1 mm (xenograft from Tutoplast® process - Tutogen Medical GmbH, Erlangen, Germany), and Bio-Oss® granules of 0.25-1 mm (anorganic xenograft - Geist-lich Pharma AG, Wolhousen, Switzerland). The Treated grafts were kindly prepared by Industrie Biomediche Insubri SA (Mezzovico-Vira, Switzerland) in ISO 13485:2016 compliant GLP-facilities. The Treated grafts were produced in blocks of 10x10x10 mm$^3$ or chips of 10x10x4 mm$^3$.

*Preparation of samples for testing:*

The sintered graft (from now on Sintered) was prepared by sintering the Treated grafts for 1 h in a furnace at 1100 °C (HTC-08/16, Nabertherm GmbH, Bremen, Germany). For cell testing, individual chips of Treated and Sintered grafts were packed in double pouching and sterilized using electron beam irradiation. For recording of FTIR, XRD, and TEM diffraction patterns, the samples (with exception of Bio-Oss) were ground into fine powder using a Mini-Mill PULVER-ISETTE 23 (FRITCH, Idar-Oberstein, Germany) ball mill.

*Attenuated total reflectance - Fourier transform infrared Spectroscopy*

ATR-FTIR spectra of the samples were collected on a Nicolet™ iS50 FTIR spectrometer (Varian Inc., Palo Alto, California, USA) with the built-in ATR module. The spectra were collected in the mid-infrared range between 400 and 4000 cm$^{-1}$ at a resolution of 4 cm$^{-1}$ by co-adding 64 scans. Background spectrum with no sample in the infrared beam was acquired before the collection of the sample spectrum. Then, the background spectrum was subtracted from the sample spectrum.

*Thermogravimetric Analysis and Differential Scanning Calorimetry*

Simultaneous thermal analyzer TGA/DSC 3$_+$ (METTLER TOLEDO, USA) was used for TGA/DSC analysis of the samples. Approximately 45-60 mg of the sample was loaded into an alumina crucible and heated from 30 to 1100 °C at a rate of 10 °C/min under flowing air (10 ml/min).

*X-ray diffraction*

XRD patterns of the samples were recorded on a Malvern Panalytical Aeris (Malvern Panalytical, United Kingdom) XRD operated at 40 kV and 15 mA (Cu K$\alpha$ radiation). Diffraction data were collected in a 10-70° 2$\theta$ range, with a step size of 0.04°. The total measurement time for each sample was 20 min.

*Micro Computed Microscopy*

MicroCT was conducted with a SkyScanner 1172 (Bruker-microCT, Kontich, Belgium) using a pixel resolution of 8.21 $\mu$m, and the X-ray source set at 64 kV and 148 $\mu$Amp, corresponding to a power of 9 W. A 360° scan was conducted with 0.53° steps, using an average of 3 frames per step. The reconstruction was done with SkyScan NRecon (Bruker-microCT, Kontich, Belgium). For the reconstruction, a ring artefact reduction of 8 was used, along with a beam hardening correction of 60%. The signal was threshold between 0.02 and 0.75. Lastly, the post-processing was defined in CTan (Bruker-microCT, Kontich, Belgium) and executed in the batch processing software BatMan. For the post-processing definition, a volume of interest (VOI) was chosen of 9743 px$^3$. From the processing 3D morphological properties were derived along with the pore accessibility.

*Scanning and Transmission Electron Microscopy*

The surface morphology of the samples was investigated using scanning electron microscopy (SEM; Hitachi Analytical tabletop SEM TM3030, Hitachi, Japan). Samples were scanned at 15 kV and x100 magnitude without using any coatings to be feasible to visually separate between mineral content and soft matter (fat and connective tissues etc). The SEM images were also coupled with Energy Dispersive X-ray Spectroscopy (EDS, Bruker, MA, US; mag. x500) to understand the local atomic composition. After the EDS analysis, the samples were sputter coated with Au for an improved SEM image acquisition. Granulated samples were investigated using Transmission Electron microscopy (TEM; JEOL JEM-2100F, Tokyo, Japan) microscope with a Schottky field emission gun (FEG) operated at 200 kV. The TEM images and diffraction patterns were acquired using a Hatan Orius 200D CCD (Gatan, Pleasantan, USA) camera, providing information about the nanoscale structure and crystallinity.

*Compressive testing*

The apparent modulus (i.e. modulus of the porous structure treated as a homogenous material [19]) and yield point (using 0.2%-$\epsilon$ offset method) were characterized using a servo-hydraulic MTS testing machine (MTS 858 Systems Inc., Minneapolis, MN, USA) equipped with a 15 kN load cell. For the Sintered samples it was necessary to change the calibration of the load cell to a maximum of 1.5 kN.

The cube samples (10x10x10 mm$^3$) were placed between two flat plates, where the bottom one was attached to a ball joint to compensate for the cube surfaces potentially being unparallel.

The samples were compressed at a rate of 1 mm/min till past the yielding point, as observed by the force level dropping or plateauing.

*Cytotoxicity:*

The cytotoxicity was measured using the exudates of the graft material. To prepare the exudates for cytotoxicity testing, the graft materials were incubated for 48 hours at 37 °C in cell medium (MEM with 15% serum and 1% antibiotic) at a concentration of 50 mL per gram of bone graft material. An osteoblastic cell line (MC3T3-E1) was seeded at a concentration of 2000 cells per well in a 96-well plate with 200 $\mu$L cell medium with 8 repeats of each group. After being allowed to attach for 24 h in an incubator, the cell medium was removed and replaced with the exudate cell medium. The 50

μL of each extract was added to a new 96-well plate and mixed with the 50 μL of a dye and catalyst mix from a LDH cytotoxicity kit (Roche Diagnostics, Indianapolis, IN, USA). After 30 minutes of incubation at 37 °C, the intensity was read off at 490 nm. The positive control had been created by adding 1% of Triton X-100 one hour prior to the extraction. The cytotoxicity from the LDH assay was calculated according to formula (1):

$$Cytotoxicity\ (\%) = \frac{I_s - I_{nc}}{I_{pc} - I_{nc}} \times 100$$

Where $I_s$ is the intensity of the sample, $I_{nc}$ is the intensity of the negative control, and $I_{pc}$ is the intensity of the positive control. Since exudate medium was used, the intensities were normalized by subtracting the mean intensity of the blank cell medium from $I_{nc}$, $I_{pc}$ and $I_s$ was normalized by subtracting the mean intensity of the blank sample exudate.

*Data processing and statistics*

All data was analysed with custom Python (Python 3.9), unless otherwise specified (e.g. μCT, SEM, and TEM). The scripts depended on the libraries including Pandas, Numpy, Matplotlib, SciPy, sklearn, Seaborn, and statannotations. Statistical differences were calculated using non-parametric Mann-Whitney U tests. A p-value below 0.05 was considered statistically significant.

**Results**

In this example, we investigated the physicochemical properties of xeno bone grafts prepared either using a low-temperature chemical treatment (referred to as "Treated", referring to decellularized xenograft obtained by the cold-cleaning process according to the present invention) and one prepared using subsequent sintering (referred to as "Sintered"). The objective was to understand the properties of the Treated graft and compare it to the state-of-the-art bone grafts. To achieve this, we performed a comprehensive physicochemical characterization using various techniques such as FTIR, XRD, TGA, TEM, and compressive testing. Furthermore, we conducted cytotoxicity testing on sterilized graft material using an LDH assay.

To make our research clinically relevant, we compared the Treated grafts against a version that was subsequently sintered and against clinically available bone grafts, namely an allograft (BTM®), a xenograft (Tutogen), and a sintered, inorganic xenograft (Bio-Oss®). This comparison allowed us to assess the suitability and potential advantages of our developed bone grafts in relation to existing commercial options.

*Physio-chemical characterization*

ATR-FTIR was used to identify characteristic absorptions related to specific chemical bonds (Fig. 1). At around 550 and 1000 cm$^{-1}$ we observed the absorption bands of the $v_3$ and $v_1$ PO$_4^{3-}$, and at 1420-1490 and 870 cm$_{-1}$ are the bands characteristic of carbonate vibrations. The phosphate bands were observable for all the grafts; however, the carbonate absorption bands were not observable for the Sintered graft. At 1550-1650 cm$^{-1}$ the band associated with N-H bending vibration of primary amines can be observed. This is not observed for Bio-Oss® nor the Sintered graft. The bands at 2900 and 2950 cm$^{-1}$ are reported to be the aliphatic C-H stretches of residual fat, and the band at 1744 cm$^{-1}$ is the v(C=O) stretching of the carboxyl in fatty acids molecules [9]. The fat-related absorption bands are evident in the untreated control sample but not in any of the other samples.

All the non-sintered samples had similar XRD patterns with clear peaks matching that of hydroxyapatite reference peaks (Fig. 1B). Also, the peaks of the sintered samples (Sintered, Bio-Oss®) matched the reference peaks, however having narrower peaks with higher intensity and peaks, suggesting that the sintering increases the crystallinity of the sample. Interestingly, the Sintered sample had peaks with smaller full width at half maximum than Bio-Oss, and we were able to demonstrate that it was possible to increase the crystallinity of Bio-Oss further. The Sintered graft and Bio-Oss® also had a peak at 37.3 degrees (Fig. 1B), which can be caused by calcination of calcium carbonate to calcium oxide [20]. From the TGA we observed a large mass decrease of approx. 40 wt.% for the Treated and 50 wt.% for the untreated bone (after mechanical debridement of bone marrow). If we consider the first derivative, we can observe the first peak at exactly 100 °C for Treated, which corresponds to the removal of water. The water content seems to be 10 wt.% for the untreated bone and 12 wt.% for Treated, suggesting the presence of collagen that can hold water. The untreated bone has a wider peak, which can be due to slower heat transfer caused by an insulating lipid layer. A second sharp peak can be found at approx. 375 °C, which is believed to be collagen [21]. In DSC results an exothermic process with heat release can be observed between 350-550 °C, suggesting combustion of the collagen. The peak of the untreated bone is taller than that of the Treated which suggests a higher collagen content. For the untreated bone there are also two shoulders to the peak, which can be theorized to belong to the lipids of the residual fat, observed with FTIR. Tutogen and BTM® followed a similar curvature to Treated, although they had a lower water peak. This is likely due to them being vacuum dried and freeze-dried respectively, meanwhile Treated has not be subjected to neither of these treatments.. Bio-Oss only exhibited a small water peak, likely due to ambient humidity, and no peak around 375 °C. This supports the observation from FTIR that Bio-Oss is inorganic, meaning that the collagen has been removed during the sintering process.

*Morphological characterization*

The morphological analysis consisted of $\mu$CT SEM and TEM, with the TEM also providing information about the crystallinity of the grafts. From the $\mu$CT it became evident that the treatment process increases the porosity of the bone, decreases the strut size, and partially increases the pore size, meanwhile, the subsequent sintering seems to increase the compactness of the graft again by reducing the pore size and the porosity (Table 1). It can also be observed that the treatment process opens the interconnectivity of the graft, while the Sintering reduces the interconnectivity at a given threshold size, when compared to the clinically available solutions, we can observe that the Treated sample exhibits a higher porosity than Tutogen, smaller struts and larger pores. Compared to BTM®, the porosity of the Treated is similar, but the strut and pore size are smaller, suggesting that Treated has more but smaller pores than BTM®. The Sintered graft has a similar porosity to Tutogen, but the strut size and pore size is significantly lower.

**Table 1:** $\mu$CT results of the bone samples before (Untreated) and after treatment (Treated) or sintering (Sintered). Benchmarked against a xenograft (Tutogen) and an allograft (BTM®). All samples were cubes of 1 cm³, from inside a volume of interest of 8x8x8 mm³ (6x6x6 mm³ for sintered due to shrinkage) was used for the morphological analysis. Bio-Oss® was not used in this study as it is only available as a fine powder. Median (IQR), n=8 (n=7 for BTM®; n=16 for Tutogen). *p-value<0.05, **p<0.01, ***p<0.001 compared to Treated, #p-value<0.05, ##p<0.01, ###p<0.001 compared to Sintered.

| Parameter | Untreated | Treated | Sintered | Tutogen | BTM® |
|---|---|---|---|---|---|
| Total Porosity (%) | 70.2 (68.5-80.8) | 80.5 (80.2-81.9) | 71.6*** (70.0-73.7) | 74.3*** (71.4-76.2) | 84.3### (80.0-86.3) |
| Object Surface /Volume (mm-1) | 18.8* (17.6-21.1) | 23.1 (20.8-25.6) | 22.6 (21.6-23.9) | 18.0***,### (17.1-18.8) | 21.9 (19.3-22.1) |
| Surface Density (mm-1) | 5.6# (4.6-6.0) | 4.3 (3.9-4.7) | 6.4*** (5.9-6.7) | 4.5### (4.1-5.5) | 3.4### (3.1-4.3) |
| Intersection Surface (mm2) | 83.0## (56.0-90.7) | 56.0 (49.2-58.6) | 45.9 (24.5-53.6) | 74.9***,### (69.4-82.9) | 45.7 (40.8-57.3) |
| Strut Thickness (μm) | 168# (154-177) | 142 (130-161) | 136 (130-139) | 182***,### (175-192) | 156 (151-169) |
| Pore Size (μm) | 473 (422-567) | 600 (573-617) | 409*** (386-450) | 593### (478-663) | 718### (610-775) |

The surface of the samples was characterized by SEM. In the untreated samples it was evident that the surface was still covered by a layer of fat. This was not a case in the remaining samples (Treated, BTM® and Tutogen). The surface of the Treated samples seems rougher and with some cracks. This can be due to the fact that surface of the Treated sample was obtained through saw cutting, meanwhile the BTM® and Tutogen samples were granules that were most likely prepared using a granulation machine. In the BTM® samples there can still be observed some strands of organic material, this is not observable in the Treated or Tutogen samples. In the Bio-Oss® and the Sintered samples there are no organic material from bone marrow observed, it all seems to have been removed. For the Sintered graft there can be observed cracks forming around the pores. Bio-Oss® is already provided in a granulated form, so it is not observable there.

The morphology and crystallinity of the samples was further investigated in TEM. The untreated sample gave limited information as the lipids most likely interfered with the signal. If we consider the non-sintered grafts (Treated, Tutogen, and BTM®) we can observe needle-like crystallites with length of a few nanometres. For the sintered graft, Bio-Oss, the crystallites are larger in the scale of tens of nanometre. Further observations can be made when evaluating the diffraction pattern from the TEM. Both the Untreated and BTM® samples have a limited diffraction pattern, which can be due to the inorganic material we can observe from the SEM. Treated, Tutogen and Bio-Oss® has clear diffraction patterns and there are indications of polycrystallinity in all samples. The Bio-Oss® sample has a spottier pattern than the other samples, which can be related to the crystallinity or to the size of the crystallites. The Sintered sample has large thick crystallites and a spot diffraction pattern typical of a highly crystalline sample, which agrees with the observations from the XRD. Meanwhile the remaining samples have more halo like patterns. The halo like patterns indicates more amorphous structures where the electrons are shattered in more directions than for a highly crystalline sample.

*Compressive strength*

The compressive testing was performed at a constant rate of 1 mm/min till past yielding (Figure 2). All groups exhibited an initial linear region, before either yielding into a non-linear, ductile or, in the case of the Sintered graft, failure in a brittle manner. From this data the apparent modulus was derived along with the yield stress and strain. In general, the data had a large variation due to the heterogenous structure of bone. It can be observed that after the cleaning process, both the apparent modulus (261 to 166 MPa) and yield stress (10.5 to 5.5 MPa) of the Treated graft have been reduced compared to the untreated sample, but neither of the differences is statistically significant. For the Sintered, the apparent modulus had been reduced to 57 MPa and it had a failure stress of 1.7 MPa, which is statistically significant both to the Treated group and untreated bone. Compared to the commercial controls the apparent modulus and yield stress of Treated is significantly lower than that of Tutogen (461 MPa & 12.1 MPa), but similar to BTM® (162 MPa & 6.9 MPa). However, already before the treatment the untreated bone had a lower modulus than Tutogen (261 vs 456 MPa), which can explain this. The Sintered graft had inferior apparent modulus and a lower failure stress than the yield stress of Tutogen and BTM®, but a comparable failure strain (2.9 $\varepsilon$%) to their yield strain (3.7 & 3.7 $\varepsilon$%). The untreated and Treated bone had the best yield strain of 4.5 and 4.9 $\varepsilon$%, which is significantly higher than for the other materials.

*Cytotoxicity and endotoxins:*

The cytotoxic effects of the exudates of the graft materials on an osteoblastic cell line (MC3T3-E1) were measured using a LDH assay. The LDH assay demonstrated the Treated samples had a lower cytotoxicity than the other groups and comparable to the negative control group. The remaining groups had a higher median approaching the 30% limit of ISO 10993-5.

**Discussion:**

In this example, we have compared a xenograft prepared after example 1, meaning a low-temperature chemical cleaning (Treated) with the graft that has further undergone sintering (Sintered), and commercial controls including a xenograft prepared using a low-temperature chemical cleaning process (Tutogen), an allograft prepared using a low-temperature chemical cleaning process (BTM®), and a sintered xenograft (Bio-Oss®). The goal was to understand the differences in physicochemical properties and in vitro biocompatibility.

A central concern for the grafts independent on the preparation process is the effective removal of bone marrow as the bone can be infected with pathogens such as viruses like HIV [22], bacteria [23], and prions [24]. Recently, there was a major outbreak of tuberculosis in the US where 113 patients received an allografts containing live cells that was infected, causing the death of 8 patients [25]. This is particularly a concern for low temperature treatment approaches, as high temperature treatment is an efficient method to inactivate or remove these pathogens but can damage the collagen structure or make the bone inorganic. For the chemical treatment, the choice of chemical steps should be considered to efficiently inactivate any pathogen. There has been reports that sodium hydroxide is efficient at removing fat from bone materials [26], however from our visual inspections of SEM images of bovine xenografts treated with either 1 M sodium hydroxide for 15 minutes or 2 h in dichloromethane, the organic solvent seems significantly more efficient at removing lipids from the graft. That said, sodium hydroxide is still essential for removing viral proteins and prions [26], but excessive exposure can compromise the graft. Based on their study, Dumas and colleagues [16] suggested that common anti-viral cleaning agents such as NaOH and $H_2O_2$ can induce deleterious changes to the mineral structure and the collagen, which *in vitro* retarded osteoblastic attachment, viability, and other biological activities. This change to the bone structure would also likely reduce the mechanical properties of the graft. From our physicochemical analysis, in particularly from the FTIR, we demonstrated that the Treated graft was free for blood, lipids and other bone marrow residuals occupying the trabecular structure of the bone. Fortunately, the mineral structure of the Treated group is not evidentially damaged or modified by the process, and the amine peaks from the collagen can be readily observed, suggesting that the final graft is a native-like organic-inorganic composite structure. For the Sintered graft, there was an observable change in the chemical structure of the bone, suggesting removal of the collagen and the carbonate structure, leaving only apatite.

For the non-sintered grafts, we observed a considerable mass decrease around 375 °C with a release of heat during TGA, which we attribute to the combustion of collagen proteins. There is also a smaller peak between 700-800 °C, which can be attributed to decomposition of calcium carbonate into calcium oxide [27]. Bio-Oss® exhibited a similar peak but at around 840 °C, meanwhile the Sintered graft had an insignificant weight change. Moreover, XRD before and after sintering of the Treated graft (yielding the Sintered graft) at 1100 °C for 60 minutes suggested that sintering significantly increased the crystallinity of the hydroxyapatite (Figure 1B). In our study we used one sintered commercial control, Bio-Oss®, although it exhibited significantly higher crystallinity than the other grafts and no collagen, the sintering at 1100 °C further increased the crystallinity. Nevertheless, in clinic Bio-Oss® has been readily observable in histology samples even 4 years after implantation [28], which is likely due to the higher crystallinity making it challenging for the body to resorb. For non-sintered grafts, e.g. SmartBone® which is a polymer coated product derived from the Tutogen graft, the remodelling occurs quickly, with clinical histology suggesting the graft is mostly resorbed after 6 months and fully resorbed after 9 months [29]. Simultaneously the SmartBone® graft successfully stimulated formation of new bone and vascularized connective tissue.

The morphological properties of the bone graft are also of significant impact on its performance. An ideal graft should have high porosity with interconnectivity to facilitate the diffusion of osteogenic cells, nutrition and removal of water products throughout the graft [2]. Our $\mu$CT results indicate that the chemical treatment of the Treated graft opens porosity and increase the interconnectivity of the graft, yielding a median porosity of over 80% and an interconnectivity of over 90% at a 200 $\mu$m cut-off threshold. This is significantly higher than for Tutogen and similar to BTM®, both being clinically successful grafts, thus, obtained results can be interpreted as clinically favourable morphological properties.

However, an increased porosity will decrease the mechanical properties due to a reduction in solid volume, meaning that it tends to be a trade-off between morphological and mechanical properties. The mechanical properties of bone grafts are of great importance as most areas require bone regeneration to have intrinsic loading, both during static situations such as standing and particularly during dynamic events such as moving (for orthopaedics) and chewing (dental). It would be catastrophic if a graft failed, as this would require invasive revision surgery. A great proportion of bone grafts are ceramic structures [30, 31] or sintered xenografts [32], where the sintering removes the organic component of the bone. A critical limitation to ceramic bone grafts is that they tend to fail in a brittle manner, making them unsuitable for load-bearing applications [33]. There are indeed methods to mitigate this risk. In orthopaedics, a surgeon would typically implant metallic fixation plates [1, 34], that can off-load the graft from the load, however, this introduces a second foreign material that is implanted, it can cause stress-shielding which can lead to asymmetric callus formation and fracture non-union [35], and it increases the risk of infection through biofilm formation on the metal surface [36]. Ideally, you would transfer as much of the loading as possible to the bone graft and limit the use of extra support devices, as the loading-induced mechanotransduction inhibits bone resorption and favours bone formation [37]. In fact, Reznikov and colleagues [38] observed in femur model in sheep that there was an inverse relation between bone graft stiffness and bone regeneration, and that this process was strain-driven in the animals that responded well to the therapy. However, they stressed major differences in the clinical response to the therapy, thus, the graft still needs long-term mechanical integrity to handle the local loading profile. In our study, we obtained comparable apparent elastic modulus and yield stress for the Treated to BTM® and somewhat lower than for Tutogen. However, the yield strain was greater for the Treated graft. Considering that the apparent modulus and yield stress were also lower for the untreated bone when compared to Tutogen, it can indicate that the starting material of Tutogen was better than the one we used, which can relate to the bovine age and race. Independently of this, the apparent modulus and yield stress are within the range of human cancellous bone (100-500 MPa and 0.1-30 MPa) [3, 39]. The compressive strengths has been proven to decrease significantly when the process temperatures surpasses 100 °C and as the process temperature is increased to 160 °C and 220 °C further collagen is removed decreasing the compressive strength [40]. The Sintered graft had significantly lower yield stress, roughly one magnitude, and the failure mechanism was different. Instead exhibiting a linear regime followed by a stress plateau as the pores collapses as is typical for cellular solids [41], it failed in a catastrophic manner with the cracks propagating through the structure. As confirmed by FTIR and TGA, there is still a significant amount of collagen left in the non-sintered grafts, which can explain their ductile resistance.

In cases where the collagen is denatured or removed through heat treatment, this will reduce the grafts' fracture toughness [42]. This clinically translates to that if a strut of a graft would fail due to local stress levels surpassing the maximum stress, the crack would likely propagate through the whole structure, which can explain the shift towards low-temperature processed grafts such as the Treated graft. As confirmed by FTIR and TGA, there is still a significant collagen content in the Treated graft, and indeed, this translates to a high yield strain in the graft. After normalizing for the solid volume of material, the Treated graft still exhibits a significantly higher yield strain than the commercial xenograft Tutogen and similar yield stress level. However, the apparent modulus was significantly lower. We theorize this is due to the starting material initially having lower mechanical stiffness than the Tutogen graft. Compared to the BTM® allograft there was no significant difference in either of the variable. Considering that both these grafts have been clinically adopted, we consider the Treated graft suitable for clinical applications. A limitation, however, is that we have not sterilized our samples prior to mechanical testing, but we are likely to do that using beta-ray sterilization, which can denature the collagen and reduce the toughness of the bone [43].

To get an early predictor of the grafts' biocompatibility, their cytotoxic effect was measured using an LDH assay. LHD measures cell death by looking at the release of lactate dehydrogenase, which is released when the plasma membrane of the cells is damaged [44]. For the LDH assay, referring to disruption of the cell membrane, the Sintered and the control grafts had a significantly higher cytotoxicity than the Treated group. Surprisingly, also the control allograft (BTM®) and xenograft (Tutogen) but prepared using low-temperature, chemical cleaning processes, exhibit significantly higher toxicity than the Treated graft. This suggests that the xenograft produced according to example 1 is likely more biocompatible than the state-of-the-art.

**Conclusion:**

The physio-chemical characterisation demonstrates that the low-temperature preparation process yields a graft comprising amorphous hydroxyapatite with the collagen maintained. This differs from the sintered grafts (Sintered and Bio-Oss®) that are highly crystalline with no collagen content, which gave significantly improved mechanical properties compared to Sintered, and the Treated grafts does not fail in a brittle manner. Compared to the allograft, BTM®, the Treated graft has a smoother stress-strain curve, suggesting fewer microfractures in the structure. Surprisingly, compared to the other bovine xenograft prepared through a cold-temperature process (Tutogen), the Treated graft had increased porosity, reduced in vitro cytotoxicity, and reached greater strain before yielding.

**Example 5:** Histological analysis of derived graft

This example is intended to demonstrate that the native organic-inorganic nature of the graft is maintained. Treated bone prepared as described in example 1 was used, and compared to untreated bovine bone and BTM®, a commercial allograft prepared using a cold-process method. Immunohistochemistry was used to detect collagen I in the samples, and the presence of osteocytes (residual cells after cleaning) in bone lacunae was investigated to confirm that the cleaning process successfully decellularized the samples.

**Method:**

*Preparation*

Samples were fixed in neutral buffered formalin 4% w/v overnight 4°C and they were decalcified in an aqueous solution containing 10% w/v EDTA pH 7.4 for 15 days at 4°C, changing solution every 3 days.

*Paraffin-embedding and sectioning*

Specimens are dehydrated in a graded series of ethanol up to the absolute ethanol, clarified in Xylene and incubated in liquid paraffin at 60°C for 2 h. Finally, samples were embedded in paraffin in a metal mold to obtain a block ready to cut by standard microtome. All samples were fully sectioned obtaining 8 $\mu$m thick sections and they were collected on glass slide, 1 section each 160 $\mu$m, in order to analyze cleaning treatment effect in all thickness;

*Immunohistochemistry (IHC)*

Sections were deparaffinized in xylene and rehydrated in absolute ethanol and distilled water. Quenching of endogenous peroxidases was performed by incubation with 0.6% $H_2O_2$ in methanol 20 min in the dark. In order to block specific binding sites, samples were incubated with 5% Goat Serum diluted in 1X PBS for 20 min at 37°C. Sections were incubated with primary antibody rabbit polyclonal anti-bovine collagen type I (GTX 44085, Genetex) diluted in 0.1% BSA/1X PBS in moist chamber at 4°C overnight; tested dilution: 1:50, 1:100; 1:200; 1:500; 1:1000, 1:2000. Next day, specimens were incubated with goat anti-rabbit biotinylated secondary antibodies (Vektor Lab) diluted 1:200 in 1.5% goat or horse serum-1X PBS solution for 60 min and then with streptavidin (Vectastain Elite ABC Kit Standard, Vektor Lab) for 30 min. In order to reveal the reaction, the sections were incubated in the substrate-chromogen solution (0.5 mg/mL 3,3 min-diaminobenzidine tetrahydrochloride activated by $H_2O_2$) (Amresco) for 5 min in the dark, counter-stained with Haematoxylin 5 min and washed in tap water 5 min to reveal the staining. Finally, the sections were dehydrated in absolute ethanol, clarified in xylene and mounted with coverslip by DPX mounting medium. The results were observed with a Nikon Eclipse Ci microscope equipped with a digital camera.

**Results:**

Treated, Uncleaned, and BTM® chips were analysed for the presence of bovine collagen type I (1:200 dilution) and for the presence of cell nuclei in bone lacunae. Results showed low/medium positivity (+, ++) in uncleaned bovine bone, the Treated bone showed low positivity (+), while BTM® showed weak positivity (-/+). No osteocytes were observed in the bone lacunae of the Treated bone, nor in the BTM® samples, while they were present in the uncleaned bone.

**Conclusion:**

The treated graft prepared from Example 1 maintains most of the collagen of the samples, confirming that it maintains its inorganic-organic nature. Compared to BTM® (human allograft) more of the collagen is maintained during the preparation process. It was also confirmed that the method successfully decellularize the graft.

**Example 6:** Use as a medical implant for treatment of bone defects

The biological material prepared in Example 1 or 2 can be sterilised and used as a medical device for tissue defect reconstruction. The sample can either be in granular form or in block form, meaning a solid block with designed geometrical dimensions. The sample is implanted to fill a naturally occurring or surgically created bone tissue void and stimulated cell migration and proliferation, thereby inducing osteoconductive bone formation. With time, the graft will be remodelled and thereby be replaced with native bone.

**Example 7:** Use as an intermediate material in the development of a reinforced medical device

The biological material prepared in either example 1 or example 2 can be used as an intermediate material to obtain a reinforced medical device. This can be done by covering the biological structure with a reinforcing layer of bioresorbable polymers with or without the inclusion of biomolecules such as gelatine before it is sterilised. A details description of the reinforcement process can be found in WO2010070416A1. This yields a medical device with improved mechanical strength compared to a medical device described in example 4, with improved cell attachment if gelatine is integrated.

**Example 8:** Use as a medical device for replacement of damaged cartilage

The biological material prepared in Example 3 can be sterilised and used as a medical device for replacement of damaged articular cartilage. After sterilisation, the material could be implanted in humans and/or animals as an implant to replace articular cartilage damaged through wear and/or osteoarthritis. How these osteochondral plugs can be implanted in human joints has been demonstrated ex vivo in figure 5. First a cylindrical void must be made, for instance with a power tool, before the implant is inserted into the created void. The osseous part of the implant will with time integrate with the surrounding native bone as described in example 6, while the cartilage will provide a low frication surface that allows smooth joint movement. It is expected that this would provide long term pain relief and improved mobility in patients that would otherwise be partly or fully disabled due to osteoarthritis.

**REFERENCES**

1. Ferracini, R., et al., Composite xenohybrid bovine bone-derived scaffold as bone substitute for the treatment of tibial plateau fractures. Applied Sciences, 2019. 9(13): p. 2675-2675.
2. Haugen, H.J., et al., Bone grafts: which is the ideal biomaterial? Journal of Clinical Periodontology, 2019. 46: p. 92-102.
3. de Lacerda Schickert, S., et al., Pre-clinical evaluation of biological bone substitute materials for application in highly loaded skeletal sites. Biomolecules, 2020. 10(6): p. 883-883.
4. Council of the European Union, Medical Device Regulation 2017/745. 2017.
5. European Commission, COMMISSION REGULATION (EU) No 722/2012. 2012.
6. European Directorate for the Quality of Medicines & HealthCare, 5.2.8 Minimizing the risk of transmitting animal spongiform encephalopath agents via human and veterinary medicinal products, in European Pharmacopoeia. 2019.
7. Reznikov, N., et al., Fractal-like hierarchical organization of bone begins at the nanoscale. Science, 2018. 360(6388): p. eaao2189-eaao2189.
8. Stevens, M.M., Biomaterials for bone tissue engineering. Materials Today, 2008. 11(5): p. 18-25.
9. Ratnayake, J.T.B., et al., Development and characterization of a xenograft material from New Zealand sourced bovine cancellous bone. Journal of Biomedical Materials Research Part B: Applied Biomaterials, 2017. 105(5): p. 1054-1062.
10. Coraça-Huber, D.C., et al., Effect of storage temperature on gentamicin release from antibiotic-coated bone chips. Cell and tissue banking, 2013. 14(3): p. 395-400.
11. Coraça-Huber, D.C., et al., Effect of two cleaning processes for bone allografts on gentamicin impregnation and in vitro antibiotic release. Cell and tissue banking, 2013. 14(2): p. 221-229.
12. Putzer, D., et al., The Influence of Liquids on the Mechanical Properties of Allografts in Bone Impaction Grafting.

Biopreservation and biobanking, 2017. 15(5): p. 410-416.

13. Putzer, D., et al., Platelet concentrate as an additive to bone allografts: a laboratory study using an uniaxial compression test. Cell and tissue banking, 2018. 19(4): p. 559-567.

14. Putzer, D., et al., The mechanical stability of allografts after a cleaning process: comparison of two preparation modes. The Journal of arthroplasty, 2014. 29(8): p. 1642-1646.

15. Kamra, P., et al., Effect of antibiotic impregnation time on the release of gentamicin from cryopreserved allograft bone chips: an in vitro study. Cell and tissue banking, 2019. 20(2): p. 267-273.

16. Dumas, A., et al., The influence of processes for the purification of human bone allografts on the matrix surface and cytocompatibility. Biomaterials, 2006. 27(23): p. 4204-4211.

17. Schoepf, C., The Tutoplast® Process: a review of efficacy. Zimmer Dental, 2008. 17: p. 40-50.

18. Bansal, M., S. Bhagat, and D. Shukla, Bovine cancellous xenograft in the treatment of tibial plateau fractures in elderly patients. International orthopaedics, 2009. 33(3): p. 779-784.

19. Ghouse, S., et al., The Design and In Vivo Testing of a Locally Stiffness-Matched Porous Scaffold. Appl Mater Today, 2019. 15: p. 377-388.

20. Tangboriboon, N., R. Kunanuruksapong, and A. Sirivat, Preparation and properties of calcium oxide from egg-shells via calcination. Materials Science-Poland, 2012. 30: p. 313-322.

21. Pourjavadi, A. and M. Kurdtabar, Collagen-based highly porous hydrogel without any porogen: Synthesis and characteristics. European Polymer Journal, 2007. 43(3): p. 877-889.

22. Alexaki, A. and B. Wigdahl, HIV-1 infection of bone marrow hematopoietic progenitor cells and their role in trafficking and viral dissemination. PLoS pathogens, 2008. 4(12): p. e1000215.

23. Binte Atique, F., R. Khalil, and M. Masudur, The bacterial contamination of allogeneic bone and emergence of multidrug-resistant bacteria in tissue bank. BioMed Research International, 2014. 2014.

24. Takakura, Y., et al., Bone marrow stroma cells are susceptible to prion infection. Biochemical and biophysical research communications, 2008. 377(3): p. 957-961.

25. Schwartz, N.G., et al., Nationwide tuberculosis outbreak in the USA linked to a bone graft product: an outbreak report. The Lancet Infectious Diseases, 2022. 22(11): p. 1617-1625.

26. Bi, L., et al., Effects of sodium hydroxide, sodium hypochlorite, and gaseous hydrogen peroxide on the natural properties of cancellous bone. Artificial organs, 2013. 37(7): p. 629-636.

27. Karunadasa, K.S., et al., Thermal decomposition of calcium carbonate (calcite polymorph) as examined by in-situ high-temperature X-ray powder diffraction. Journal of Physics and Chemistry of solids, 2019. 134: p. 21-28.

28. Piattelli, M., et al., Bone reactions to anorganic bovine bone (Bio-Oss) used in sinus augmentation procedures: a histologic long-term report of 20 cases in humans. International Journal of Oral and Maxillofacial Implants, 1999. 14(6): p. 835-840.

29. D'Alessandro, D., et al., Bovine bone matrix/poly (L-lactic-co-$\varepsilon$-caprolactone)/gelatin hybrid scaffold (Smart-Bone®) for maxillary sinus augmentation: A histologic study on bone regeneration. International journal of pharmaceutics, 2017. 523(2): p. 534-544.

30. Haugen, H., et al., Ceramic TiO2-foams: characterisation of a potential scaffold. Journal of the European Ceramic Society, 2004. 24(4): p. 661-668.

31. Jelusic, D., et al., Monophasic $\beta$-TCP vs. biphasic HA/$\beta$-TCP in two-stage sinus floor augmentation procedures-a prospective randomized clinical trial. Clinical oral implants research, 2017. 28(10): p. e175-e183.

32. Xu, A.T., et al., The optimization of sintering treatment on bovine-derived bone grafts for bone regeneration: in vitro and in vivo evaluation. Journal of Biomedical Materials Research Part B: Applied Biomaterials, 2020. 108(1): p. 272-281.

33. Laurencin, C., Y. Khan, and S.F. El-Amin, Bone graft substitutes. Expert Review of Medical Devices, 2006. 3(1): p. 49-57.

34. Ferracini, R., et al., Bone Loss in Distal Radial Fractures Treated with A Composite Xenohybrid Bone Substitute: A Two Years Follow-Up Retrospective Study. Materials (Basel), 2020. 13(18): p. 4040-4040.

35. Beltran, M.J., C.A. Collinge, and M.J. Gardner, Stress modulation of fracture fixation implants. Journal of the American Academy of Orthopaedic Surgeons, 2016. 24(10): p. 711-719.

36. Goodman, S.B., et al., The future of biologic coatings for orthopaedic implants. Biomaterials, 2013.

37. Oftadeh, R., et al., Biomechanics and mechanobiology of trabecular bone: a review. Journal of biomechanical engineering, 2015. 137(1): p. 10802-10802.

38. Reznikov, N., et al., Individual response variations in scaffold-guided bone regeneration are determined by independent strain- and injury-induced mechanisms. Biomaterials, 2019. 194: p. 183-194.

39. Munford, M.J., K.G. Ng, and J.R. Jeffers, Mapping the multi-directional mechanical properties of bone in the proximal tibia. Advanced Functional Materials, 2020. 30(46): p. 2004323.

40. Abdelmoneim, D., et al., The effect of low-processing temperature on the physicochemical and mechanical properties of bovine hydroxyapatite bone substitutes. Materials, 2022. 15(8): p. 2798.

41. Gibson, L.J., Biomechanics of cellular solids. Journal of Biomechanics, 2005. 38(3): p. 377-399.

42. Burton, B., et al., Bone embrittlement and collagen modifications due to high-dose gamma-irradiation sterilization. Bone, 2014. 61: p. 71-81.

43. Kaminski, A., et al., Effect of accelerated electron beam on mechanical properties of human cortical bone: influence of different processing methods. Cell and tissue banking, 2012. 13: p. 375-386.

44. Kumar, P., A. Nagarajan, and P.D. Uchil, Analysis of cell viability by the lactate dehydrogenase assay. Cold Spring Harbor Protocols, 2018. 2018(6): p. pdb. prot095497.

**Claims**

1. A cleaning process to produce a decellularized bone tissue sample or decellularized osteochondral tissue sample, comprising cleaning steps, each step combining mechanical and chemical cleaning treatments, carried out at standard pressure and a low temperature, of a biological material sample, said process comprising of:

   a) Providing a biological material sample selected from the group comprising bone tissue sample/s or osteochondral tissue sample/s, as a shaped block/s, or chip/s, respectively;
   b) Treating said biological material sample/s at a series of cleaning steps at standard pressure and low temperature, preferably no more than 37 °C, each step combining mechanical and chemical cleaning treatments, as follows:

   i. Soaking said biological material sample/s into distilled water as a chemical cleaning treatment in combination with centrifugation and sonication of said biological material sample as a mechanical cleaning treatments;
   ii. Soaking biological material sample/s treated according to step bi) into Slightly alkaline solution (pH=8.0-10.0) as a chemical cleaning treatment in combination with centrifugation and sonication of said biological material sample/s as a mechanical cleaning treatments;
   iii. Soaking biological material sample/s treated according to step bii) into organic solvent as a chemical cleaning treatment in combination with centrifugation and sonication of said biological material sample/s as a mechanical cleaning treatments;
   iv. Soaking biological material sample/s treated according to step biii) into Alkaline solution (pH=10.0-14.0) as a chemical cleaning treatment in combination with centrifugation and sonication of said biological material sample/s as a mechanical cleaning treatments;
   v. Reacting biological material sample/s treated according to step biv) with Oxidating agent as a chemical cleaning treatment in combination with centrifugation and sonication of said biological material sample as a mechanical cleaning treatments.

2. Process according to claim 1, wherein the low temperature is from 20°C to 45°C, preferably from 23 °C to 42 °C., more preferably no more than 37°C.

3. Process according to claims 1 to 2, wherein the organic solvent is selected from the group comprising: Polar aprotic solvent, such as DCM, Acetone, Acetonitrile, ethyl acetate, pyridine,tetrahydrofuran; halogen-alkane, such as Dichloroethane, Trichloroethane, Chloroform, Carbon tetrachloride, Carbon disulfide ; alcohol, such as Ethanol, Propanol, Methanol, Butanol; ether, such as Diethyl ether; alkane, such as Pentane, Hexane.

4. Process according to claims 1 to 3, wherein the Oxidating agent is a solution of a oxidating reagent selected from the group comprising: $H_2O_2$, Peracetic acid, $Na_2O_2$ $MgO_2$, $CaO_2$, $K_2O_2$ $Na_2CO_3$

5. Process according to claims 1 to 4 wherein the centrifugation is for at least 1 minute and less than 20 minutes, at a relative centrifugal force of at least 3000xg and less than 9000xg.

6. Process according to claims 1 to 4 wherein the mechanical cleaning treatment, as a solid-liquid extraction treatment is selected from centrifugation extraction, sonication, water jet, hydraulic pressure, super critical fluid extraction.

7. Process according to claims 1 to 6 wherein the bone tissue sample/s or decellularized osteochondral tissue sample/s is/are of animal origin, such as bovine, equine, and porcine, or from humans both living and cadavers.

8. A decellularized bone tissue sample or decellularized osteochondral tissue sample obtainable, preferably obtained, by the process according to any one of the claims 1 to 7.

9. A decellularized bone tissue sample or decellularized osteochondral tissue sample obtainable, preferably obtained, by the process according to any one of the claims 1 to 7 and subsequently washed, dried and sterilized for use as a medical device.

10. A decellularized bone tissue sample obtainable, preferably obtained, by the process according to any one of the claims 1 to 7 and subsequently washed, dried and sterilized for use as a base matrix of a bone implant matrix.

11. A decellularized bone tissue sample or decellularized osteochondral tissue sample obtainable, preferably obtained, by the process according to any one of the claims 1 to 7 and subsequently washed, dried and sterilized for use as a xenograft/allograft material for filling of bone voids and to induce bone regeneration in dental and orthopaedic bone defects.

12. A decellularized bone tissue sample or decellularized osteochondral tissue sample according to claim 11, wherein the dental bone defects relates to the jaw and other osseotissues in the oral orifice.

13. A decellularized bone tissue sample or decellularized osteochondral tissue sample according to claim 11, wherein the orthopaedic bone defects relates to all bone tissue defects including the skull and flat bone, long bone, short bone, and vertebrae/vertebras.

14. A decellularized bone tissue sample or decellularized osteochondral tissue sample obtainable, preferably obtained, by the process according to any one of the claims 1 to 7 and subsequently washed, dried and sterilized for use as a xenograft/allograft material to induce ectopic bone formation, preferably in a spinal fusion procedure and/or ankle fusion procedures.

15. A decellularized osteochondral tissue sample obtainable, preferably obtained, by the process according to any one of the claims 1 to 7 and subsequently washed, dried and sterilized for use as a xenograft/allograft material to replace damages cartilage in joints including the knees, hip, elbow, shoulders, wrists, fingers, toes, and facet joints, as well as replacing cartilage at the end of vertebrae/vertebras, with the osseous phase of the osteochondral graft having the ability to integrate with the surrounding native bone tissue.

Fig. 1

Fig. 2

Fig. 3

Fig. 4

Fig. 5

Europäisches
Patentamt

European
Patent Office

Office européen
des brevets

# EUROPEAN SEARCH REPORT

**Application Number**

EP 24 17 3587

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 977 432 A (WOLFINBARGER JR LLOYD [US] ET AL) 2 November 1999 (1999-11-02) <br> * column 4, lines 15-64 * <br> * column 7, line 44 - column 9, line 64 * <br> * page 12, line 65 - page 13, line 48 * <br> * examples * <br> * claims * | 1-8, 10-14 | INV. <br> A61L27/36 |
| X | DE 10 2014 102503 A1 (TUTOGEN MEDICAL GMBH [DE]) 27 August 2015 (2015-08-27) | 8 | |
| A | * page 3, paragraph 8 * <br> * page 4, paragraph 11 - page 6, paragraph 27 * <br> * examples * <br> * claims * | 1-7,9-15 | |
| X | WO 2012/141454 A2 (HANS BIOMED COR [KR]; HWANG HO CHAN [KR] ET AL.) 18 October 2012 (2012-10-18) | 8,9,15 | |
| A | * page 3, paragraph 23 * <br> * example 1 * <br> * claims * | 1-7, 10-14 | TECHNICAL FIELDS SEARCHED (IPC) |
| | | | A61L |
| X | CN 115 414 531 A (SHANGHAI YAPENG BIOLOGICAL TECH CO LTD) 2 December 2022 (2022-12-02) | 8 | |
| A | * examples * <br> * claims * | 1-7,9-15 | |
| A | CN 114 209 883 B (TIANXINFU BEIJING MEDICAL EQUIPMENT STOCK LTD COMPANY) 23 August 2022 (2022-08-23) <br> * examples 1-3 * <br> * claims * | 1-15 | |

The present search report has been drawn up for all claims

| Place of search | Date of completion of the search | Examiner |
|---|---|---|
| The Hague | 16 September 2024 | Van den Bulcke, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 3587

16-09-2024

| Patent document cited in search report | | Publication date | Patent family member(s) | | Publication date |
|---|---|---|---|---|---|
| US 5977432 | A | 02-11-1999 | NONE | | |
| DE 102014102503 | A1 | 27-08-2015 | DE 102014102503 A1 | | 27-08-2015 |
| | | | EP 3110246 A1 | | 04-01-2017 |
| | | | KR 20160127077 A | | 02-11-2016 |
| | | | WO 2015128097 A1 | | 03-09-2015 |
| WO 2012141454 | A2 | 18-10-2012 | NONE | | |
| CN 115414531 | A | 02-12-2022 | NONE | | |
| CN 114209883 | B | 23-08-2022 | NONE | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010070416 A1

### Non-patent literature cited in the description

- **FERRACINI, R. et al.** Composite xenohybrid bovine bone-derived scaffold as bone substitute for the treatment of tibial plateau fractures. *Applied Sciences*, 2019, vol. 9 (13), 2675-2675
- **HAUGEN, H.J. et al.** Bone grafts: which is the ideal biomaterial?. *Journal of Clinical Periodontology*, 2019, vol. 46, 92-102
- **DE LACERDA SCHICKERT, S. et al.** Pre-clinical evaluation of biological bone substitute materials for application in highly loaded skeletal sites. *Biomolecules*, 2020, vol. 10 (6), 883-883
- *Medical Device Regulation 2017/745*, 2017
- *COMMISSION REGULATION (EU) No 722/2012*, 2012
- 5.2.8 Minimizing the risk of transmitting animal spongiform encephalopath agents via human and veterinary medicinal products. *European Pharmacopoeia*, 2019
- **REZNIKOV, N. et al.** Fractal-like hierarchical organization of bone begins at the nanoscale. *Science*, 2018, vol. 360 (6388), eaao2189-eaao2189
- **STEVENS, M.M.** Biomaterials for bone tissue engineering. *Materials Today*, 2008, vol. 11 (5), 18-25
- **RATNAYAKE, J.T.B. et al.** Development and characterization of a xenograft material from New Zealand sourced bovine cancellous bone. *Journal of Biomedical Materials Research Part B: Applied Biomaterials*, 2017, vol. 105 (5), 1054-1062
- **CORAÇA-HUBER, D.C. et al.** Effect of storage temperature on gentamicin release from antibiotic-coated bone chips. *Cell and tissue banking*, 2013, vol. 14 (3), 395-400
- **CORAÇA-HUBER, D.C. et al.** Effect of two cleaning processes for bone allografts on gentamicin impregnation and in vitro antibiotic release. *Cell and tissue banking*, 2013, vol. 14 (2), 221-229
- **PUTZER, D. et al.** The Influence of Liquids on the Mechanical Properties of Allografts in Bone Impaction Grafting. *Biopreservation and biobanking*, 2017, vol. 15 (5), 410-416

- **PUTZER, D. et al.** Platelet concentrate as an additive to bone allografts: a laboratory study using an uniaxial compression test. *Cell and tissue banking*, 2018, vol. 19 (4), 559-567
- **PUTZER, D. et al.** The mechanical stability of allografts after a cleaning process: comparison of two preparation modes. *The Journal of arthroplasty*, 2014, vol. 29 (8), 1642-1646
- **KAMRA, P. et al.** Effect of antibiotic impregnation time on the release of gentamicin from cryopreserved allograft bone chips: an in vitro study. *Cell and tissue banking*, 2019, vol. 20 (2), 267-273
- **DUMAS, A. et al.** The influence of processes for the purification of human bone allografts on the matrix surface and cytocompatibility. *Biomaterials*, 2006, vol. 27 (23), 4204-4211
- **SCHOEPF, C.** The Tutoplast® Process: a review of efficacy. *Zimmer Dental*, 2008, vol. 17, 40-50
- **BANSAL, M. ; S. BHAGAT ; D. SHUKLA**. Bovine cancellous xenograft in the treatment of tibial plateau fractures in elderly patients. *International orthopaedics*, 2009, vol. 33 (3), 779-784
- **GHOUSE, S. et al.** The Design and In Vivo Testing of a Locally Stiffness-Matched Porous Scaffold. *Appl Mater Today*, 2019, vol. 15, 377-388
- **TANGBORIBOON, N. ; R. KUNANURUKSAPONG ; A. SIRIVAT**. Preparation and properties of calcium oxide from egg-shells via calcination. *Materials Science-Poland*, 2012, vol. 30, 313-322
- **POURJAVADI, A. ; M. KURDTABAR**. Collagen-based highly porous hydrogel without any porogen: Synthesis and characteristics. *European Polymer Journal*, 2007, vol. 43 (3), 877-889
- **ALEXAKI, A. ; B. WIGDAHL**. HIV-1 infection of bone marrow hematopoietic progenitor cells and their role in trafficking and viral dissemination. *PLoS pathogens*, 2008, vol. 4 (12), e1000215
- **BINTE ATIQUE, F. ; R. KHALIL ; M. MASUDUR**. The bacterial contamination of allogeneic bone and emergence of multidrug-resistant bacteria in tissue bank. *BioMed Research International*, 2014, vol. 2014

- **TAKAKURA, Y. et al.** Bone marrow stroma cells are susceptible to prion infection. *Biochemical and biophysical research communications*, 2008, vol. 377 (3), 957-961
- **SCHWARTZ, N.G. et al.** Nationwide tuberculosis outbreak in the USA linked to a bone graft product: an outbreak report. *The Lancet Infectious Diseases*, 2022, vol. 22 (11), 1617-1625
- **BI, L. et al.** Effects of sodium hydroxide, sodium hypochlorite, and gaseous hydrogen peroxide on the natural properties of cancellous bone. *Artificial organs*, 2013, vol. 37 (7), 629-636
- **KARUNADASA, K.S. et al.** Thermal decomposition of calcium carbonate (calcite polymorph) as examined by in-situ high-temperature X-ray powder diffraction. *Journal of Physics and Chemistry of solids*, 2019, vol. 134, 21-28
- **PIATTELLI, M. et al.** Bone reactions to anorganic bovine bone (Bio-Oss) used in sinus augmentation procedures: a histologic long-term report of 20 cases in humans. *International Journal of Oral and Maxillofacial Implants*, 1999, vol. 14 (6), 835-840
- **D'ALESSANDRO, D. et al.** Bovine bone matrix/poly (L-lactic-co-ε-caprolactone)/gelatin hybrid scaffold (SmartBone®) for maxillary sinus augmentation: A histologic study on bone regeneration. *International journal of pharmaceutics*, 2017, vol. 523 (2), 534-544
- **HAUGEN, H. et al.** Ceramic TiO2-foams: characterisation of a potential scaffold. *Journal of the European Ceramic Society*, 2004, vol. 24 (4), 661-668
- **JELUSIC, D. et al.** Monophasic β-TCP vs. biphasic HA/β-TCP in two-stage sinus floor augmentation procedures-a prospective randomized clinical trial. *Clinical oral implants research*, 2017, vol. 28 (10), e175-e183
- **XU, A.T. et al.** The optimization of sintering treatment on bovine-derived bone grafts for bone regeneration: in vitro and in vivo evaluation. *Journal of Biomedical Materials Research Part B: Applied Biomaterials*, 2020, vol. 108 (1), 272-281
- **LAURENCIN, C.** ; **Y. KHAN** ; **S.F. EL-AMIN**. Bone graft substitutes. *Expert Review of Medical Devices*, 2006, vol. 3 (1), 49-57
- **FERRACINI, R. et al.** Bone Loss in Distal Radial Fractures Treated with A Composite Xenohybrid Bone Substitute: A Two Years Follow-Up Retrospective Study. *Materials (Basel)*, 2020, vol. 13 (18), 4040-4040
- **BELTRAN, M.J.** ; **C.A. COLLINGE** ; **M.J. GARDNER**. Stress modulation of fracture fixation implants. *Journal of the American Academy of Orthopaedic Surgeons*, 2016, vol. 24 (10), 711-719
- **GOODMAN, S.B. et al.** The future of biologic coatings for orthopaedic implants. *Biomaterials*, 2013
- **OFTADEH, R. et al.** Biomechanics and mechanobiology of trabecular bone: a review. *Journal of biomechanical engineering*, 2015, vol. 137 (1), 10802-10802
- **REZNIKOV, N. et al.** Individual response variations in scaffold-guided bone regeneration are determined by independent strain- and injury-induced mechanisms. *Biomaterials*, 2019, vol. 194, 183-194
- **MUNFORD, M.J.** ; **K.G. NG** ; **J.R. JEFFERS**. Mapping the multi-directional mechanical properties of bone in the proximal tibia. *Advanced Functional Materials*, 2020, vol. 30 (46), 2004323
- **ABDELMONEIM, D. et al.** The effect of low-processing temperature on the physicochemical and mechanical properties of bovine hydroxyapatite bone substitutes. *Materials*, 2022, vol. 15 (8), 2798
- **GIBSON, L.J.** Biomechanics of cellular solids. *Journal of Biomechanics*, 2005, vol. 38 (3), 377-399
- **BURTON, B. et al.** Bone embrittlement and collagen modifications due to high-dose gamma-irradiation sterilization. *Bone*, 2014, vol. 61, 71-81
- **KAMINSKI, A. et al.** Effect of accelerated electron beam on mechanical properties of human cortical bone: influence of different processing methods. *Cell and tissue banking*, 2012, vol. 13, 375-386
- **KUMAR, P.** ; **A. NAGARAJAN** ; **P.D. UCHIL**. Analysis of cell viability by the lactate dehydrogenase assay. *Cold Spring Harbor Protocols*, 2018, vol. 2018 (6)